# NEUE EUROPÄISCHE PATENTSCHRIFT

(11) **EP 0 908 086 B2**
(45) Veröffentlichungstag und Bekanntmachung des Hinweises auf die Entscheidung über den Einspruch: **07.06.2006**
(45) Hinweis auf die Patenterteilung: 08.01.2003
(21) Anmeldenummer: 98117585.4
(22) Anmeldetag: 16.09.1998
(51) Int. Cl.: A01D 41/12

(54) **Vorrichtung zur Feuchtemessung in Erntemaschinen**
Device for moisture measuring of grain in harvesting machines
Dispositif de mesure de l'humidité de grain dans des machines de récolte

(30) Priorität: 09.10.1997 DE 19744485
(43) Veröffentlichungstag der Anmeldung: 14.04.1999
(73) Patentinhaber: CLAAS Selbstfahrende Erntemaschinen GmbH, 33428 Harsewinkel (DE)
(72) Erfinder: Behnke, Willi, 33803 Steinhagen (DE); Diekhans, Norbert, Dr., 33335 Gütersloh (DE); Eggenhaus, Georg, 48346 Ostbevern (DE); Wesselmann, Winfried, 48231 Warendorf (DE)

(56) Entgegenhaltungen:
- EP-A- 0 042 245
- EP-A- 0 804 872
- CA-A- 2 182 989
- DE-A- 1 300 316
- DE-A- 3 232 746
- JP-A- 56 074 648
- JP-A- 56 128 449
- US-A- 5 092 819
- US-A- 5 616 851

## Beschreibung

Die vorliegende Patentanmeldung betrifft eine Erntemaschine mit Feuchtemesseinrichtung sowie eine Vorrichtung zur Feuchtemessung in Erntemaschinen, bestehend aus Mitteln zur Entnahme des Meßgutes aus einem Erntegutstrom, zur Zuführung des Meßgutes in einen Meßraum, einem Meßraum, einem zugehörigen Feuchtigkeitssensor, und Mitteln zur Rückführung des Meßgutes in einen Erntegutstrom.

Zur Optimierung des Anbaus von Feldfrüchten und die den wirklichen Ertordemissen angepaßte Ausbringung von Dünge- und Pflanzenschutzmitteln ist es wichtig zu wissen, welche Ertragsmengen auf jeweiligen Teilen der Anbaufläche erzielt wurden. Um aus diesen Werten Aussagen für die nachfolgenden Anbaufrüchte gewinnen zu können, sollten die gemessenen Ertragswerte des Erntegutes möglichst präzise sein. Der Ertrag wird dabei aus dem Gutstrom in der Erntemaschine berechnet, der wiederum auf die Masse des lagerfähig getrockneten Erntegutes zu beziehen ist. Um diese Ertragskorrektur positionsgenau durchführen zu können, ist schon während der Ernte eine genaue Bestimmung der Gutfeuchte erforderlich. Auch können aus der gemessenen Feuchte des Erntegutes Werte zur Einstellung von Komponenten einer Erntemaschine abgeleitet werden, was ebenfalls eine hohe Präzision der Meßwerte erforderlich macht. Eine gattungsgemäße Vorrichtung ist beispielsweise aus der DE 41 05 857 bekannt. Die dort gezeigte Vorrichtung zur Feuchtemessung dient dazu, einen von einer ersten kapazitiven Meßeinrichtung ermittelten Mengenmeßwert durch einen von einer zweiten Meßeinrichtung ermittelten Feuchtemeßwert zu korrigieren. Die zweite Meßeinrichtung besteht aus einem Überlaufgefäß, dem in einem Zeitintervall durch eine Öffnung mehr Meßgut zugeführt wird als durch eine untenseitige Öffnung entweichen kann. Dadurch ist unter normalen Emtebedingungen eine ausreichende Befüllung der Meßeinrichtung bei kontinuierlichem Austausch des anfallenden Meßgutes sichergestellt.

Eine andere Feuchtemeßeinrichtung ist aus der US 5,616,851 bekannt. Dort wird eine Einrichtung vorgeschlagen, bei der zur Befüllung der Meßeinrichtung eine Steuerklappe nur solange in einer "offen"-Stellung verbleibt, bis ein Sensor die ausreichende Befüllung der Meßvorrichtung meldet. Beiden Meßeinrichtungen ist gemeinsam, daß sie jeweils einen Nebenstrom aus dem laufenden Gutstrom für Meßzwecke ableiten und nach erfolgter Messung die entnommene Erntegutmenge wieder dem Hauptgutstrom zuführen. Beiden Einrichtungen sind die Nachteile gemeinsam daß sie leicht verschmutzen, wodurch die Meßmengen und werte verfälscht werden, und Verstopfungen auftreten können. Da die Sensoren während der laufenden Erntearbeit nicht ständig kontrolliert werden können, ist es möglich, daß aufgrund der Verschmutzung oder Verstopfung des Meßraumes oder des Feuchtigkeitssensors über einen längeren Zeitraum verfälschte Werte gemessen werden. Die falschen Werte sind für nachfolgende Auswertungen unbrauchbar. Da oftmals nachträglich nicht festgestellt werden kann, ab welchem Zeitpunkt die ermittelten Meßwerte fehlerhaft sind, müssen häufig nicht nur die tatsächlich fehlerhaften, sondern oft auch eigentlich korrekte Werte aufgegeben werden. Da in vielen Regionen nur einmal im Jahr geerntet wird, bedeutet ein solcher Datenverlust einen erheblichen Rückschlag in dem Bemühen, eine verläßliche Datenbasis fürteilschlagspezifische Anwendungen aufzubauen, zumal auch in Folgejahren abermals Datenverluste auftreten können. Noch größer jedoch kann der Schaden sein, wenn fehlerhafte Daten ermittelt werden, die nachfolgend als korrekt angenommen für Auswertungszwecke weiterverarbeitet werden. Solche unbemerkt fehlerhaften Daten können bei der nachfolgenden Planung des Einsatzes von Saatgut, Dünge- und Planzenschutzmitteln zu schwerwiegenden Fehlallokationen führen, die sich nachteilig auf den Anbauerfolg des Landwirtes auswirken. Aus diesen Gründen ist es wichtig, die Feuchtigkeitsmessung möglichst genau durchzuführen und alle möglichen Fehlerquellen sicher zu eliminieren.

Demgemäß ist es die Aufgabe der vorliegenden Erfindung, die Vorrichtung zur Feuchtemessung in Erntemaschinen sowie eine Erntemaschine mit Feuchtemesseinrichtung zu verbessern.

Die Aufgabe wird durch die kennzeichnenden Merkmale des Anspruchs 1 oder 16 gelöst. Bevorzugt werden Mittel vorgesehen, die sowohl den Meßraum als auch zumindest einen Feuchtigkeitssensor zwangsentleeren und/oder zwangsreinigen. Das Merkmal des Zwangsentleerens oder des Zwangsreinigens bedeutet, daß mindestens das Entleeren oder das Reinigen zwangsweise geschieht, indem entweder durch die Form des Mittels oder durch die Taktung des Mittels eine Entleerung oder Reinigung bewirkt wird. Die Zwangsentleerung und/oder Zwangsreinigung von Meßraum und Feuchtigkeitssensor stellt sicher, daß immer wieder ein Meßwert mit neuem Meßgut bestimmt werden kann, da der Meßraum nicht mehr verstopft ist. Auch wird die Gefahr geringer, daß der Sensor zur Messung der Füllung des Meßraumes verschmiert und so eine ständiger Füllung des Meßraumes simulier wird oder am Feuchtigkeitssensor oder den Wänden des Meßraumes anhaftendes altes Erntegut den Meßwert für den aktuellen Ernteguststrom verfälscht. Insgesamt Wird durch die vorgeschlagenen Mittel die Zuverlässigkeit der Feuchtemessung und damit die Genauigkeit und Verwertbarkeit der ermittelten Feuchtewerte deutlich erhöht.

Weitere vorteilhafte Ausgestaltungen der vorliegenden Erfindungen ergeben sich aus den kennzeichnenden Merkmalen der Unteransprüche, auf die insoweit verwiesen wird.

Die Erfindung wird nun anhand von mehreren Ausführungsbeispielen näher erläutert. Es zeigen:
- Figur 1:: einen Körnerelevator eines Mähdreschers in einer Ansicht, wobei seitlich am Körnerelevator eine Vorrichtung zur Feuchtemessung angebracht ist,
- Figur 2:: eine weitere Ansicht des in Figur 1 dargesteltten Körnerelevators mit angebauter Feuchtemeßeinrichtung,
- Figur 3:: eine detaillierte Darstellung der Vorrichtung zur Feuchtemessung,
- Figur 4:: eine Schnittdarstellung aus einer Sicht von oben entlang der Linie IV-IV in Figur 3,
- Figur 5:: eine Schnittdarstellung entlang der Linie V-V in Figur 3,
- Figur 6:: eine Schnittdarstellung entlang der Linie VI-VI in Figur 3,
- Figur 7:: eine alternative Ausführungsform einer Vorrichtung zur Feuchtemessung mit einem vertikal beweglichen Schieber,
- Figur 8:: eine andere Position der in Figur 7 gezeigten Zwangsentleerungs- und Reinigungsmittel,
- Figur 9, 9a:: eine alternative Ausführungsform einer Vorrichtung zur Feuchtemessung mit einem seitlich in den zu messenden Gutstrom fahrbaren Feuchtigkeitssensor,
- Figur 10:: eine alternative Ausführungsform einer Vorrichtung zur Feuchtemessung mit einem seitlich in den Meßraum fahrbaren Feuchtigkeitssensor,
- Figur 11:: eine alternative Ausführungsform einer Vorrichtung zur Feuchtemessung mit einem Zellenrad zur Bildung des MeBraumes,
- Figur 12:: eine alternative Ausführungsform einer Vorrichtung zur Feuchtemessung mit einem um eine Schwenkachse beweglichen Schieber.

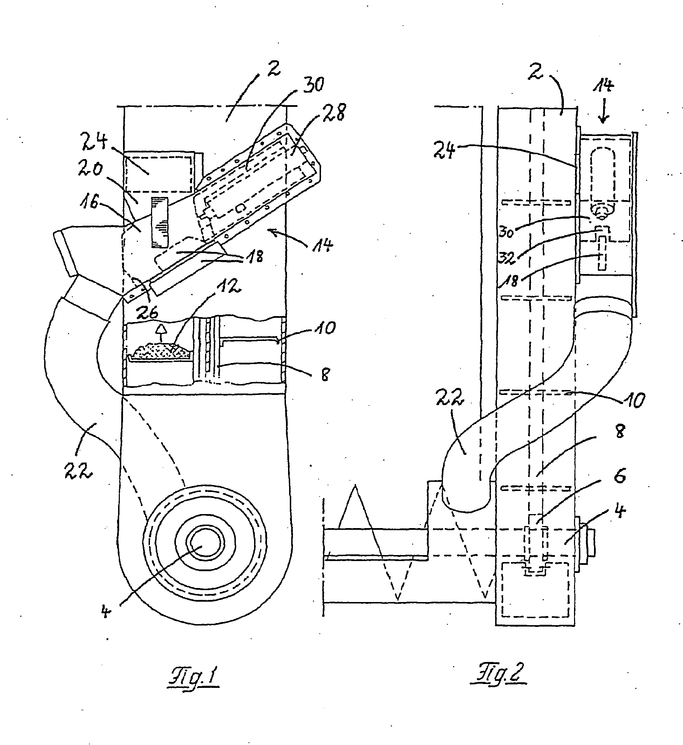

In Figur 1 ist ein Körnerelevator 2 eines nicht näher dargestellten Mähdreschers gezeigt, in dem eine Kette 8 mit daran befestigten Förderplatten 10 um auf Wellen 4 aufgesetzte Zahnräder 6 umläuft. Die Förderplatten 10 nehmen zugefördertes Erntegut 12 auf und fördern es zu einer entfernt liegenden Abgabestation, wo es weiterbefördert oder zwischengelagert wird. Dort geben die Förderplatten 10 das Erntegut 12 ab und laufen zur Aufgabestation zurück, um erneut eine-Portion Erntegut 12 aufzunehmen. Die Abfolge einer Vielzahl von den Förderplatten 10 geförderten Portionen von Erntegut 12 bildet einen Erntegutstrom, dessen Gehalt an Feuchtigkeit von einer Vorrichtung zur Feuchtemessung 14 ermittelt werden kann. Die Vorrichtung zur Feuchtemessung 14 besteht aus einem Meßraum 16, einem Feuchtigkeitssensor 18, einem Zuführkanal 20 und einem Rückführkanal 22. Das Erntegut 12, dessen Feuchte bestimmt werden soll, gelangt durch eine seitliche Öffnung 24 in der Wand des Körnerelevators 2 zunächst in den Zuführkanal 20 und rieselt durch diesen hindurch in den Meßraum 16. Der Meßraum 16 ist durch eine Schließklappe 26 verschlossen. Nach einer bestimmten Zeit füllt sich der Meßraum 16 soweit auf, daß der Feuchtigkeitssensor 18 eine zuverlässige Messung des Feuchtigkeitsgehaltes des im Meßraum 16 angesammelten Ernteguts 12 durchführen kann. Nach Beendigung der Messung fährt ein Hydraulikzylinder 28 als Stellorgan einen Schieber 30 vor, der das angesammelte Erntegut 12 zunächst gegen die Schließklappe 26 drückt und diese dadurch aufstößt. Das Erntegut 12 kann den Meßraum verlassen. Bei Vorfahrt des Schiebers 30 bis in seine maximale Ausfahrstellung wird der Meßraum 16 6 vollständig entleert. Die Schließklappe 26 kann eine Geometrie aufweisen, durch die die Schließklappe 26 beim Vorfahren des Schiebers 30 auf dessen Vorderseite Verunreinigungen oder anhaftendes Erntegut 12 abstreift.

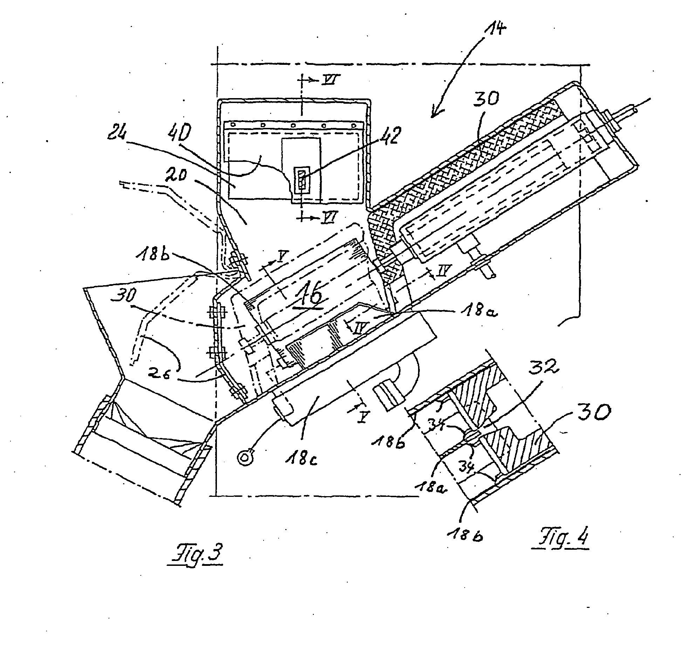

Wie in Figur 2 gezeigt ist, weist der Schieber 30 eine Aussparung 32 auf, deren Form annähernd an die Form des Feuchtigkeitssensors 18 angepaßt ist. Beim Überfahren des Feuchtigkeitssensors 18 durch den Schieber 30 streifen die seitlich an den Feuchtigkeitssensor 18 heranreichenden Flächen des Schiebers 30 an dem Feuchtigkeitssensor 18 anhaftende Ansammlungen von Erntegut 12 oder sonstigen Verschmutzungen ab. Auf diese Weise wird der Feuchtigkeitssensor 18 sowie auch der Meßraum 16 insgesamt gereinigt. Beim Zurückfahren des Schiebers ist dieser wieder bereit zur Vornahme einer erneuten Messung. Das ausgestoßene Erntegut kann durch den Rückführkanal 22 wieder dem Erntegutstrom zugerührt werden. Wie In Figur2 gezeigt, kann die Zuführung beispielsweise von oben auf eine Querförderschnecke erfolgen.

Die Betätigung des Schiebers 30 kann abhängig vom Eintritt verschiedener Bedingungen erfolgen. Als Bedingung kann beispielsweise eine manuelle Schaltung des Schiebers 30 erfolgen. Es kann aber auch eine nicht näher dargestellte Steuerungselektronik den Schieber30 zeittakt-, durchsatz-, emtegutabhängig oder aus einer beliebigen Kombination dieser Parameter betätigen. Eine ebenfalls nicht näher dargestellte Regelungselektronik kann eine Zykluszelt nach Bedarf optimieren. Auch ist es denkbar, die Öffnung des Meßraums 16 so lange zu verzögern, bis das bereits gemessene Meßgut nicht wieder in den Meßraum 16 gelangen kann, weil es schon weggefördert ist. Alternativ kann auch so schnell gemessen werden, daß das bereits gemessene Meßgut den Meßraum noch nicht erreicht hat. Es ist vortellhaft, den Meßwert des Feuchtigkeitssensors 18 nach Entleerung beziehungsweise Reinigung des Meßraums 16 auf einen geeigneten Offsetwert zu setzen, da Abweichungen vom geeigneten Offsetwert bei der nachfolgenden Messung einen entsprechenden Fehler bedeuten würden. So kann es sinnvoll sein, den Offsetwert auf den Meßwert der Feuchtigkeit in Umgebungsluft, beispielsweise 7 %, zu setzen.

Figur 3 zeigt eine detailliertere Darstellung der Vorrichtung zur Feuchtemessung 14. Im Meßraum 16 ist der Feuchtigkeitssensor 18 angeordnet. Im Ausführungsbeispiel besteht er aus einem kapazitiven Sensor, er kann wahlweise jedoch auch aus anderen aus dem Stand der Technik bekannten Sensoren bestehen, die dazu in der Lage sind, einen Feuchtigkeitswert von einem Erntegut 12 zu bestimmen. Als alternative Meßmethoden zur Bestimmung der Feuchtigkeit seien hier neben der Kapazität/Leitfähigkeit beispielhaft die Ausgleichsfeuchte, der Gasdruck, die mechanische oder elektronische Infrarottrocknung, die Mikrowellen-Absorption, -resonanz oder -trocknung, Die NIR-Spektroskopie, die NMR-Spektroskopie oder die Thermo-Elektrolyse genannt. Der beispielhafte kapazitive Feuchtigkeitssensor 18 besteht aus einer ersten Elektrode 18 a, die etwa mittig elektrisch von den Seitenwänden des Meßraumes 16 isoliert im Meßraum 16 steht. Zwischen der Elektrode 18 a und einer Gegenelektrode 18 b, die im Ausführungsbeispiel flächig an der Seitenwand des Meßraumes 16 angeordnet ist, besteht das elektrische Meßfeld. Wenn das Gehäuse des Meßraums 16 aus einem nichtleitenden Material wie beispielsweise Kunststoff besteht, muß eine separate Elektrode 18 b angebracht werden. Wenn das Gehäuse des Meßraums 16 aus einem elektrisch leltenden Material besteht, genügt es, das Gehäuse unter Spannung zu setzen. Unterhalb des Meßraumes 16 ist die Meßelektronik in einem Gehäuse 18 c angeordnet. Zusätzlich zum Feuchtigkeitswert kann die Meßelektronik im Gehäuse 18 c zusätzliche relevante Parameter ermitteln, wie beispielsweise die Temperatur des Ernteguts 12, die einen Einfluß auf den gemessenen Feuchtigkeitswert hat.

Der Schieber 30 ist auf seiner Oberseite so ausgebildet, daß er mit seiner der Öffnung 24 zugewandten Seite in seiner maximalen Ausfahrstellung den Meßraum 16 gegen nachströmendes Erntegut 12 abdichtet. Die Bewegung der Schließklappe 26 kann passiv vom Schieber 30 über das Erntegut 12 auf die Schließklappe 26 übertragen werden. Gegebenenfalls kann die Schließklappe noch mit einer Federkraft beaufschlagt sein, die die Schließklappe 26 wieder in ihre Ausgangsstellung zurückbewegt. Es kann jedoch auch ein mit dem Schieber 30 oder dem Hydraulikzylinder 28 verbundenes, nicht näher dargestelltes Hebelgestänge eine Bewegung auf die Schließklappe 26 übertragen. Die Schließklappe 26 kann so weit anhebbar sein, daß sie eine Kontrolle und Reinigung des Rückführkanals 22 ermöglicht. Das Füllvolumen des Zuführkanals 20 sollte bei durch den Schieber 30 verschlossenem Meßraum 16 nicht größer sein als das Füllvolumen des Meßraums 16. Zwar würde ein größeres Füllvolumen eine schnelle Wiederbefüllung des Meßraumes 16 ermöglichen, allerdings ist zu beachten, daß der Druck von zusätzlichem sich über dem Meßraum 16 befindlichem Erntegut 12 den Feuchtigkeitsmeßwert beeinflussen kann. Auch ist zu beachten, daß eine zu große Ansammlung von Erntegut nicht mehr den wahren Meßwert für das aktuell geerntete Erntegut wiedergibt, sondern den Meßwert für Emtegut, das schon kurz nach dem letzten Entleer- bzw. Reinigungszyklus des Schiebers 30 angesammelt worden ist. Für die Vergleichbarkeit der ermittelten Meßwerte sollte eine möglichst konstante Befüllung des Meßraumes sichergestellt sein. Ergeben sich wegen schwankender Durchsatzmengen unterschiedliche Schütthöhen im Zuführkanal 20, so können sich bei eigentlich gleichem Feuchtigkeitsgehaft unterschiedliche Meßwerte ergeben. Auch ist es vorteilhaft, wenn der aktuelle Neigungswinkel der Erntemaschine, der die Schütthöhe im Zuführkanal 20 beeinflussen kann, bei der Ermittlung des Feuchtigkeitswertes von der Auswerteelektronik mitberücksichtigt werden kann.

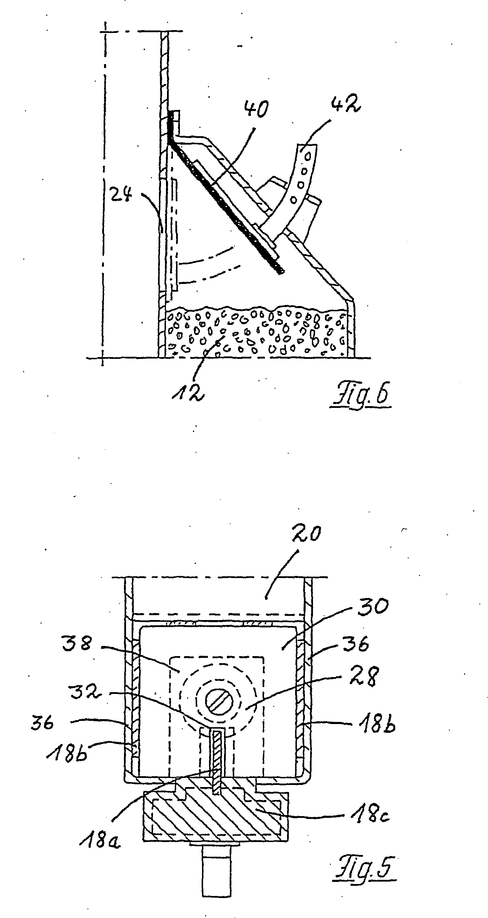

In Figur 4 ist gut erkennbar, wie der Schieber 30 mit seiner Aussparung 32 bei einer Vorwärtsbewegung über die Elektrode 18 a hinweggleiten kann. Mit seinen Seitenflächen gleitet der Schieber 30 über die Elektroden 18 b. Der Schieber 30 weist Abstreifmittel 34 auf, die beispielsweise als elastische Lippen oder Bürsten ausgebildet sein können und gleichzeitig den Schieber 30 während seiner Bewegung im Meßraum 16 führen. Die Elektroden 18a, 18b dienen auf diese Weise als Leitmitel für den Schieber 30. Die Abstreifmittel 34 streifen nicht nur Erntegut 12 und Verschmutzungen von den Elektroden 18 a, 18 b ab, sondern verhindert auch, daß die Flächen des Schiebers 30 seitlich über die Oberfläche der Elektroden 18 a, 18 b schleifen und auf diese Weise die Elektroden beschädigt bzw. abgeschliffen werden. Die nach innen hin angestellten seitlichen Flächen der Aussparung 32 erleichtern die seitliche Ausrichtung des Schiebers 30 bei seiner Rückfahrbewegung, wen sie mit der Elektrode 18 a in Kontakt kommen.

In Figur 5 ist im Querschnitt durch den Meßraum 16 die Stirnfläche des Schiebers 30 zu erkennen, die mit der Aussparung 32 über die Elektrode 18 a gleitet. Seitlich grenzt der Schieber 30 an die Elektroden 18 b an, die auf den Seitenwänden 36 der Vorrichtung zur Feuchtemessung 14 angebracht sind. Oberhalb des Schiebers 30 ist der Zuführkanal 20 angedeutet. Der Körper des Schiebers 30 umschließt einen Hohlraum 38, in dem der Hydraulikzylinder 28 angeordnet ist.

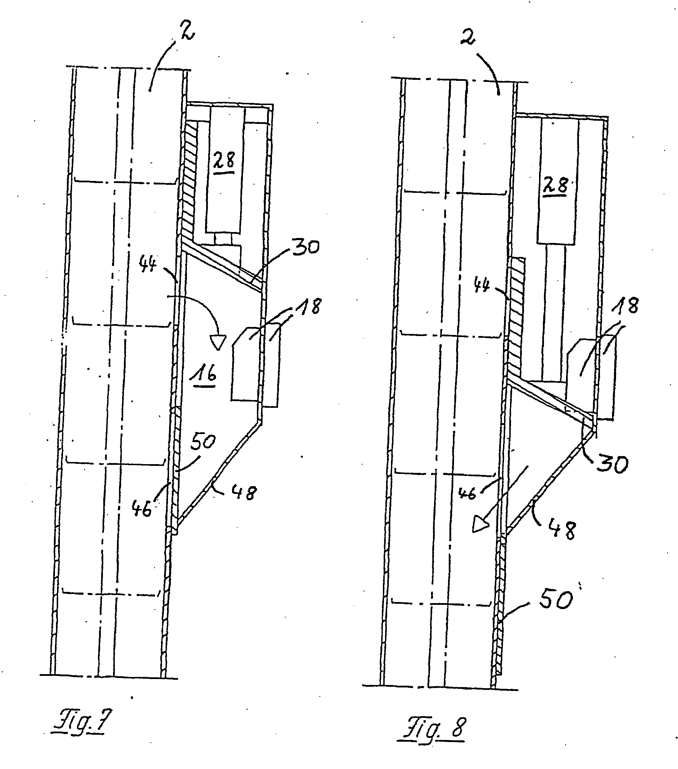

Figur 6 zeigt eine verschwenkbare Verschlußklappe 40, mittels der die seitliche Öffnung 24 in der Seitenwand des Körnerelevators 2 verschließbar ist. Die Verschwenkung erfolgt mittels eines Schwenkhebels 42, der manuell oder motorisch betätigbar ist. Die Verschlußklappe kann - beispielsweise auch zusammen mit dem Hydraulikzylinder 28 über ein Hebelgestänge - betätigt werden, um zwecks Erzielung einer gleichmäßigen Meßportion eine überfüllte Menge von Erntegut 12 abzustreichen und zurück in der Körnerelevator 2 zu drängen. Es kann aber auch sinnvoll sein, den Feuchtigkeitssensor 18 zu verschließen, beispielsweise, wenn keine Feuchtigkeitsmessung gewünscht ist und ein unnötiger Verschleiß des Sensors vermieden werden soll. In einer bevorzugten Ausgestaltung sollte der Schieber 30 stillgesetzt sein, wenn die Verschlußklappe 40 geschlossen ist.

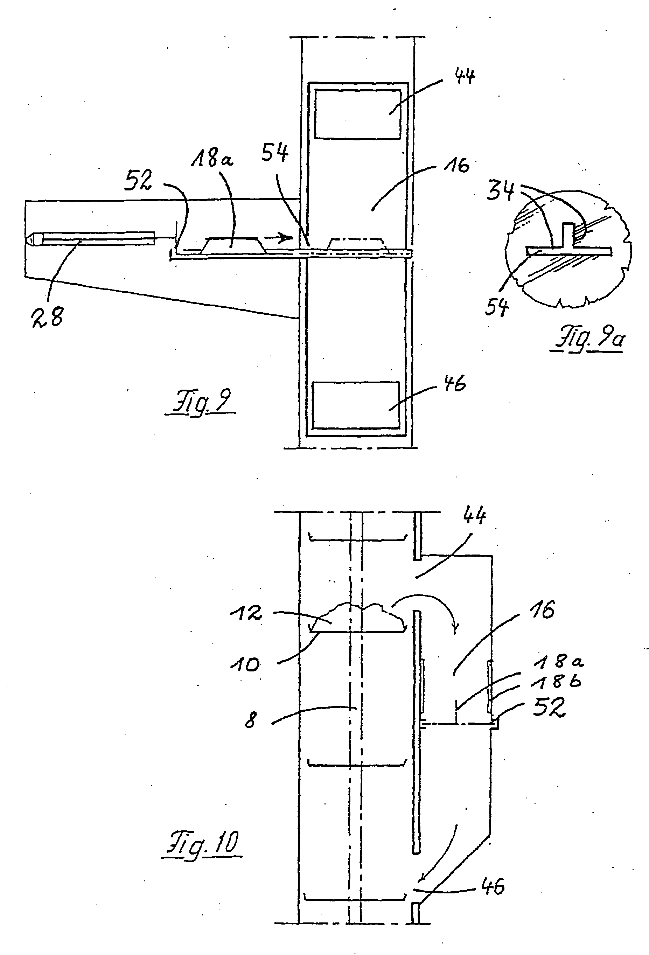

In den Figuren 7 und 8 ist eine alternative Ausführungsform dargestellt, in der der Meßraum 16 seitlich am Körnerelevator 2 so angeordnet ist, daß das Meßgut nach Bestimmung des Feuchtigkeitswertes durch den Feuchtigkeitssensor 18 direkt in den Körnerelevator 2 zurückgefördert wird. Um mit einem Stelimittel gleichzeitig die Zuführöffnung 44 und die Ausführöffnung 46 trotz feststehender Seitenwand 48 zu öffnen und zu verschließen, ist der Schieber 30 mit einer Verschlußlasche 50 fest verbunden, die so angeordnet ist, daß die Verschlußlasche 50 in der am weitesten zurückgezogenen Stellung des Schiebers 30 die Ausführöffnung 46 verschließt. In dieser Stellung des Schiebers 30 ist die Zuführöffnung 44 offen, sodaß im Meßraum 16 eine Portion Meßgut angesammelt werden kann. Nach erfolgter Messung überfährt der Schieber 30 mit seiner Aussparung 32 den Feuchtigkeitssensor 18 in bereits beschriebener Weise. Dabei wird die Verschlußlasche 50 vom Schieber 30 aus dem Meßraum 16 herausbewegt, wodurch sich die Ausführöffnung 46 öffnet und das Meßgut in den Körnerelevator abfließen kann. Der Schieber 30 verschließt dabei die Zuführöffnung 44 und verhindert so, daß neues Meßgut in den Meßraum 16 eindringen kann: Erst, wenn der Schieber 30 wieder in eine zurückgezogene Position bewegt ist, kann neues Meßgut in den Meßraum 16 durch die Zuführöffnung 44 hineinströmen.

Anstelle der in den Figuren 7 und 8 gezeigten Ausführung kann natürlich auch eine Anordnung gewählt werden, bei der die Ausführöffnung 46 kleiner ist als die Zuführöffnung 44, sodaß der Meßraum 16 ständig befüllt ist und wegen Fehlens der Verschlußiasche 50 ein kontinuierlicher Austausch des Meßgutes gewährleistet ist. Der Schieber 30 wird in einersolchen Anordnung nurzur Reinigung und gelegentlichen vollständigen Entleerung des Meßraums 16-bzw. des Feuchtigkeitssensors 18 betätigt, um die kontinuierliche Ermittlung des Feuchtigkeitswertes nicht zu beeinträchtigen.

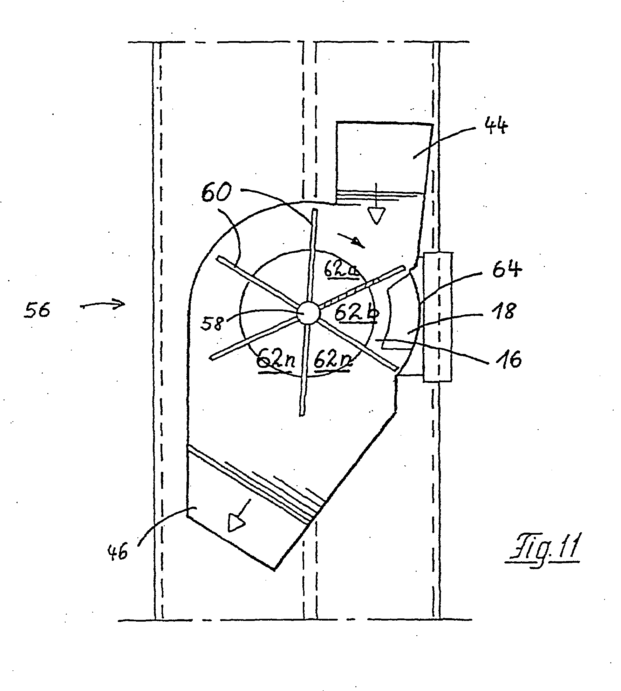

In den Figuren 9, 9a und 10 ist ein Ausführungsbeispiel gezeigt, in dem die Elektrode 18 a auf einem Schlitten 52 angeordnet ist. Der Schlitten 52 wird durch einen Hydraulikzylinder 28 betätigt in den Meßraum 16 ein-und wieder herausgefahren. Wenn der Schlitten 52 in den Meßraum 16 eingefahren ist, wird das durch die Zuführöffnung 44 in den Meßraum 16 eindringende Meßgut an der Elektrode 18 a aufgestaut, und es kann eine Messung vorgenommen werden. Nach Beendigung der Messung kann der Schlitten 52 wieder aus dem Meßraum 16 herausgefahren werden, und das angestaute Meßgut kann den Meßraum 16 durch die Ausführöffnung 46 wieder verlassen. Der Schlitten 52 mit der Elektrode 18 a wird durch eine in der Seitenwand des Meßraumes 16 befindliche Öffnung 54 bewegt. Die Geometrie der Öffnung 54 ist so ausgestaltet, daß sie die äußere Kontur des Schlittens 52 beziehungsweise der Elektrode 18 a eng umschließt, wie es in Figur 9 a gezeigt ist. Dadurch wird beim Ein- und Ausfahren des Schlittens 52 eine Abstreif- und Reinigungswirkung erzielt. An der Begrenzungskante der Öffnung 54 können zusätzliche Abstreifmittel 34 angeordnet sein, deren Vorteile bereits oben näher dargestelft sind.

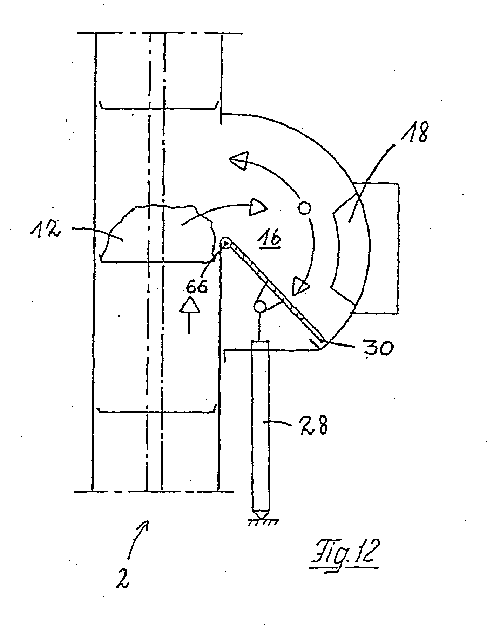

In Figur 11 ist eine Meßvorrichtung gezeigt, die mit einem Zellenrad 56 arbeitet. Das Zellen rad 56 kann motorisch angetrieben sein, oder es wird durch die Schwerkraft des zugeförderten Erntegutes in eine drehende Bewegung versetzt. Das Zellenrad 56 besteht aus einem zyllndrischen Körper, auf dessen Welle 58 in von der Welle 58 abweisender Richtung eine Mehrzahl von Zellwänden 60 aufgesetzt sind, die teilweise die zwischen sich befindlichen Zellen 62 a, 62 b, 62 n räumlich begrenzen. Durch die Zuführöffnung 44 zugefördertes Erntegut 12 fällt in die Zelle 62 a. Durch die Rotationsbewegung des Zellenrades 56 wird die Zelle 62 a in den Bereich der Wandffäche 64 bewegt, die gleichzeitig den Feuchtigkeitssensor 18 trägt und den Meßraum 16 zusammen mit den Zellwänden 60 einer Zelle 62, im Ausführungsbeispiel der Zelle 62 b, abschließt. Nach Beendigung der Messung kann das Erntegut 12 den Meßraum 16 und den Bereich des Zellenrades 56 durch die Ausführöffnung 46 verlassen. Bemerkenswert an diesem Ausführungsbeispiel ist, das die Rückführstelle in den abwärts führenden Teil des Körnerelevators 2 mündet, der regelmäßig nicht mit Erntegut befüllt ist, sodaß das Meßgut sicher abgefördert werden kann. Die Zellwände 60 weisen nicht näher dargestellte Aussparungen 32 auf, die in ihrer Geometrie auf den Feuchtigkeitssensor 18 abgestimmt sind.

In Figur 12 gelangt das Erntegut 12 in den Meßraum 16. Nach Durchführung der Messung mittels des Feuchtigkeitssensors 18 wird der Schieber 30 vom Hydraulikzylinder 28 um die feststehende Schwenkachse 66 bewegt. Dabei wird der Meßraum 16 geleert und gereinigt und das Meßgut wieder in den Körnerelevator 2 gedrückt.

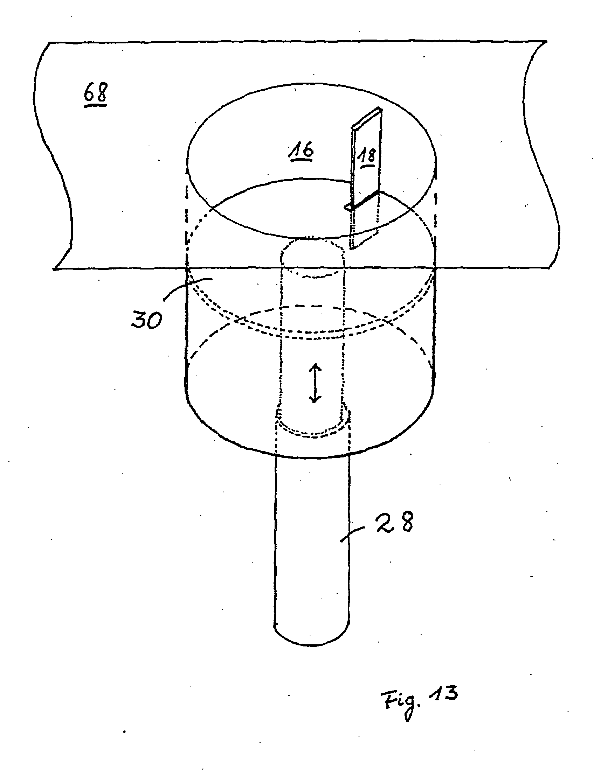

Die Erläuterung der Erfindung ist nur als beispielhaft zu verstehen dem Fachmann bereitet es keinerlei Schwierigkeiten, den Ertindungsgegenstand so anzupassen, daß er auch in anderen Fördervorrichtungen als einem Körnerelevator einsetzbar ist. So könnte die Erfindung neben der Anwendung an einem Körnerelevator vom Typ eines Kettenförderers beispielsweise auch an einer Querförderschnecke eingesetzt werden, indem an die seitliche Wandung zur Querfördererschnecke ein Meßraum 16 in Form eines Quaders oder Zylinders angesetzt wird, in dem sich ein Schieber 30 in Form eines Kolbens auf und ab bewegt, um den Meßraum zu befüllen und zu entleeren. Ein Beispiel für eine solche Anordnung findet sich in Figur 13. Diese Form eines Feuchtigkeitssensors nebst Schieber kann auch in anderen bewegten Förderelementen wie beispielsweise einem Vorbereitungs- oder Rücklaufboden eines Mähdreschers eingesetzt werden. Auch kann die Erfindung in Wurtschächten wie beispielsweise in einem Feldhäcksler oder Förderschächten einer Ballenpresse oder eines Ladewagens oder bei Grünfuttergeräten wieselbstfahrenden oder gezogenen Mähwerken, Wendem oder Schwadern eingesetzt werden. Auch hinsichtlich der beschriebenen Merkmale bereitet es dem Fachmann keine Schwierigkeiten, diese durch ihm bekannte gleichwirkende Mittel zu ersetzen oder die vorgeschlagenen Mittel für bestimmte Anwendungsfälle so abzuwandeln, daß die erfinderische Idee im konkreten Anwendungsfall einsetzbar ist.

## Patentansprüche

1. Ernemaschine mit Feuchtemesseinrichtung bestehend aus einem Messraum, aus Mitteln zur Entnahme des Messgutes aus einem Erntegutstrom und zur Zuführung des Messgutes in den Messraum, einem zugehörigen Feuchtigkeitssensor und Mitteln zur Rückführung des Messgutes in den Erntegutstrom,
**dadurch gekennzeichnet, dass**
der Messraum (16) und/oder zumindest ein Feuchtigkeitssensor (18) durch Mittel (30) zwangsgeleert und/oder zwangsgereinigt wird und wobei die Mittel (30) innerhalb des Messraums (16) bewegbar sind.

2. Vorrichtung nach Ansprüchen 1,
**dadurch gekennzeichnet,**
**daß** sowohl der Meßraum (16) als auch zumindest ein Feuchtigkeitssensor (18) durch Mittel (30) zwangsgeleert und/oder zwangsgereinigt werden.

3. Vorrichtung nach einem oder mehreren der Ansprüche 1 und 2,
**dadurch gekennzeichnet,**
**dass** der Messraum (16) mit einem darin befindlichen Feuchtigkeitssensor (18) seitlich so an einem Förderorgan der Erntemaschine angebracht ist, dass Erntegut in den Messraum (16) gelangen kann, und an einer Seite des Messraums (16) ein Schieber (30) angeordnet ist, der bei einer Bewegung den Messraum (16) zumindest teilweise entleert.

4. Vorrichtung nach Anspruch 3,
**dadurch gekennzeichnet,**
**daß** der Schieber (30) bei einer Bewegung zur zumindestteilweisen Entleerung des Meßraums (16) gleichzeitig den Feuchtigkeitssensor (18) reinigt.

5. Vorrichtung nach einem der Ansprüche 1 bis 4,
**dadurch gekennzeichnet,**
**daß** die Betätigung des Mittels (30) vom Eintritt einer Bedingung abhängig ist.

6. Vorrichtung nach einem oder mehreren der Ansprüche 1 bis 5,
**dadurch gekennzeichnet,**
**daß** das Mittel (30) durch zumindest einen Stellmotor oder Hubzylinder (28) beweglich Ist.

7. Vorrichtung nach einem oder mehreren derAnsprüche 1 bis 6,
**dadurch gekennzeichnet,**
**daß** das Mittel (30) den Meßraum (16) während einer Bewegung oder in einer Ausfahrstellung zumindest teilweise verschließt.

8. Vorrichtung nach einem oder mehreren der Ansprüche 1 bis 7,
**dadurch gekennzeichnet,**
**daß** der Meßraum (16) zumindest teilweise durch eine schwenkbeweglich angeordnete Schließklappe (26) begrenzt ist.

9. Vorrichtung nach einem oder mehreren derAnsprüche 1 bis 8,
**dadurch gekennzeichnet,**
**daß** das Füllvolumen des Zuführkanals (20) gleich oder kleiner ist als das Füllvolumen des Meßraums (16).

10. Vorrichtung nach einem oder mehreren derAnsprüche 1 bis 9,
**dadurch gekennzeichnet,**
**daß** eine Auswerteelektronik neben dem Sensorwert für die Feuchtigkeit weitere Sensorwerte zur Korrektur des Feuchtigkeitswertes berücksichtigt.

11. Vorrichtung nach einem oder mehreren derAnsprüche 1 bis 9,
**dadurch gekennzeichnet,**
**daß** das Mittel (30) in seiner Bewegung im Meßraum (16) durch Leitmittel geführt ist

12. Vorrichtung nach einem oder mehreren der Ansprüche 1 bis 11,
**dadurch gekennzeichnet,**
**daß** die Bestimmung des Feuchtigkeitswertes durch den Feuchtigkeitssensor (18) kontinuierüch oder diskontinuierlich erfolgt.

13. Vorrichtung nach einem oder mehreren derAnsprüche 1 bis 12,
**dadurch gekennzeichnet,**
**daß** das Meßgut über die Elevatorzuführschnecke dem Erntegutstrom zurückgeführt wird.

14. Vorrichtung nach einem oder mehreren der Ansprüche 1 bis 13,
**dadurch gekennzeichnet,**
**daß** der Schieber (30) um eine Schwenkachse (66) geschwenkt wird.

15. Vorrichtung nach einem oder mehreren der Ansprüche 1 bis 14,
**dadurch gekennzeichnet,**
**daß** der Boden des Förderraumes vom Meßraum (16) zur Ausführöffnung (46) zumindest bereichsweise eine Neigung von mindestens 30 bei ebener Stellung der Erntemaschine aufweist.

16. Vorrichtung zur Feuchtemessung in Emtemaschinen, bestehend aus einem Messraum, aus Mitteln zur Entnahme des Messgutes aus einem Erntegutstrom und zur Zuführung des Messgutes in den Messraum, einem zugehörigen Feuchtigkeitssensor und Mitteln zur Rückführung des Messgutes in den Erntegutstrom,
**dadurch gekennzeichnet,**
**dass** der Messraum (16) wenigstens teilweise im Erntegutsrom (12) angeordnet Ist und Wände (60) eines Zellenrades (56) den Messraum (16) zumindest teilweise begrenzen.

17. Vorrichtung nach einem oder mehreren der Ansprüche 1 bis 16,
**dadurch gekennzeichnet,**
**daß** die Rückführstelle des Meßgutes zum Erntegutstrom mit dem Meßzyklus so abgestimmt ist, daß keine oder möglichst nur eine kleine Menge des alten Meßguts wieder In den Meßraum (16) gelangt.

18. Vorrichtung nach einem oder mehreren der Ansprüche 1 bis 17,
**dadurch gekennzeichnet,**
**daß** die Rückführstelle des Meßgutes in den Abschnitt einer Fördereinrichtung (2) mündet, In dem sich regelmäßig kein oder nur wenig Erntegut (12) befindet.

19. Vorrichtung nach einem oder mehreren der Ansprüche 1 bis 18,
**dadurch gekennzeichnet,**
**daß** nach Reinigung beziehungsweise Entleerung des Meßraumes (16) der Meßwert des Feuchtigkeitssensors (18) jeweils auf einen geeigneten Offsetwert gesetzt wird.

## Claims

1. Harvester with moisture measurement apparatus, comprising a measuring chamber, means for taking the material to be measured from a flow of crop material and for feeding the material to be measured into the measuring chamber, an associated moisture sensor and means for returning the material to be measured into the flow of crop material, **characterised in that** the measuring chamber (16) and/or at least one moisture sensor (18) is positively emptied and/or positively cleaned by means (30), and wherein the means (30) are movable within the measuring chamber (16).

2. Apparatus according to claim 1 **characterised in that** both the measuring chamber (16) and also at least one moisture sensor (18) are positively emptied and/or positively cleaned by means (30).

3. Apparatus according to one or more of claims 1 and 2 **characterised in that** the measuring chamber (16) with a moisture sensor (18) disposed therein is laterally mounted to a conveyor member of the harvester in such a way that crop material can pass into the measuring chamber (16) and arranged at one side of the measuring chamber (16) is a pusher (30) which upon movement at least partially empties the measuring chamber (16).

4. Apparatus according to claim 3 **characterised in that** upon movement for at least partially emptying the measuring chamber (16) the pusher (30) simultaneously cleans the moisture sensor (18).

5. Apparatus according to one of claims 1 to 4 **characterised in that** actuation of the means (30) is dependent on the onset of a condition.

6. Apparatus according to one or more of claims 1 to 5 **characterised in that** the means (30) is movable by at least one setting motor or stroke cylinder (28).

7. Apparatus according to one or more of claims 1 to 6 **characterised in that** the means (30) at least partially closes the measuring chamber (16) during a movement or in an extension position.

8. Apparatus according to one or more of claims 1 to 7 **characterised in that** the measuring chamber (16) is at least partially delimited by a PivotTable arranged closure flap (26).

9. Apparatus according to one or more of claims 1 to 8 **characterised in that** the filling volume of the feed passage (20) is equal to or less than the filling volume of the measuring chamber (16).

10. Apparatus according to one or more of claims 1 to 9 **characterised in that** an electronic evaluation system, besides the sensor value for moisture, takes account of further sensor values for correction of the moisture value.

11. Apparatus according to one or more of claims 1 to 9 **characterised in that** the means (30) is guided by guide means in its movement in the measuring chamber (16).

12. Apparatus according to one or more of claims 1 to 11 **characterised in that** determination of the moisture value by the moisture sensor (18) is effected continuously or discontinuously.

13. Apparatus according to one or more of claims 1 to 12 **characterised in that** the material to be measured is returned to the flow of crop material by way of the elevator feed screw.

14. Apparatus according to one or more of claims 1 to 13 **characterised in that** the pusher (30) is pivoted about a pivot axis (66).

15. Apparatus according to one or more of claims 1 to 14 **characterised in that** the floor of the conveyor chamber from the measuring chamber (16) to the discharge opening (46) has at least in a region-wise manner a slope of at least 30° when the harvester is in a level position.

16. Apparatus for moisture measurement in harvesters, comprising a measuring chamber, means for taking the material to be measured from a flow of crop material and for feeding the material to be measured into the measuring chamber, an associated moisture sensor and means for returning the material to be measured into the flow of crop material, **characterised in that** the measuring chamber (16) is arranged at least partly in the crop material flow (12) and walls (60) of a cell wheel (56) at least partially delimit the measuring chamber (16).

17. Apparatus according to one or more of claims 1 to 16 **characterised in that** the return location of the material to be measured to the flow of crop material is so matched to the measuring cycle that no amount of the old material to be measured or as far as possible only a small amount thereof passes into the measuring chamber (16) again.

18. Apparatus according to one or more of claims 1 to 17 **characterised in that** the return location of the material to be measured opens into a portion of a conveyor device (2), in which there is regularly no or only a little crop material (12).

19. Apparatus according to one or more of claims 1 to 18 **characterised in that** after cleaning or emptying of the measuring chamber (16) the measurement value of the moisture sensor (18) is respectively set to a suitable offset value.

## Revendications

1. Machine de récolte équipée d'un dispositif de mesure de l'humidité du grain, comprenant une chambre de mesure, des moyens pour prélever le produit à mesurer sur un flux de produit récolté et pour amener ledit produit à mesurer dans la chambre de mesure, un détecteur d'humidité associé et des moyens pour ramener le produit à mesurer dans le flux de produit récolté, **caractérisée en ce que** la chambre de mesure (16) et/ou au moins un détecteur d'humidité (18) sont vidés et/ou nettoyés par force par des moyens (30), les moyens (30) pouvant être déplacés à l'intérieur de la chambre de mesure (16).

2. Dispositif selon la revendication 1, **caractérisé en ce qu'**aussi bien la chambre de mesure (16) qu'au moins un détecteur d'humidité (18) sont vidés et/ou nettoyés par force par des moyens (30).

3. Dispositif selon une ou plusieurs des revendications 1 et 2, **caractérisé en ce que** la chambre de mesure (16) avec un détecteur d'humidité (18) monté dans celle-ci, est fixée sur le côté d'un organe de transport de la machine de récolte de telle sorte que le produit récolté puisse entrer dans la chambre de mesure (16), et **en ce que** sur un côté de la chambre de mesure (16) est disposé un piston (30) qui, par déplacement, vide au moins partiellement la chambre de mesure.

4. Dispositif selon la revendication 3, **caractérisé en ce que** le piston (30), lors d'un déplacement pour vider au moins partiellement la chambre de mesure (16), nettoie simultanément le détecteur d'humidité (18).

5. Dispositif selon une des revendications 1 à 4, **caractérisé en ce que** l'actionnement du moyen (30) est dépendant de l'apparition d'une condition.

6. Dispositif selon une ou plusieurs des revendications 1 à 5, **caractérisé en ce que** le moyen (30) peut être déplacé par au moins un moteur de commande ou par un vérin (28).

7. Dispositif selon une ou plusieurs des revendications 1 à 6, **caractérisé en ce que** le moyen (30), pendant un déplacement ou dans une position avancée, ferme au moins partiellement la chambre de mesure (16).

8. Dispositif selon une ou plusieurs des revendications 1 à 7, **caractérisé en ce que** la chambre de mesure (16) est au moins partiellement limitée par un volet de fermeture (26) monté pivotant.

9. Dispositif selon une ou plusieurs des revendications 1 à 8, **caractérisé en ce que** le volume de remplissage du canal d'alimentation (20) est égal ou inférieur au volume de remplissage la chambre de mesure (16).

10. Dispositif selon une ou plusieurs des revendications 1 à 9, **caractérisé en ce qu'**une électronique de traitement exploite en plus de la valeur délivrée par le détecteur d'humidité, des valeurs de détecteurs supplémentaires pour la correction des valeurs d'humidité.

11. Dispositif selon une ou plusieurs des revendications 1 à 9, **caractérisé en ce que** le moyen (30), pour son déplacement à l'intérieur de la chambre de mesure (16), est guidé par des moyens de guidage.

12. Dispositif selon une ou plusieurs des revendications 1 à 11, **caractérisé en ce que** la détermination de la valeur d'humidité est réalisée de manière continue ou discontinue par le détecteur d'humidité (18).

13. Dispositif selon une ou plusieurs des revendications 1 à 12, **caractérisé en ce que** le produit à mesurer est ramené dans le flux de produit récolté au-dessus de la vis d'élévateur.

14. Dispositif selon une ou plusieurs des revendications 1 à 13, **caractérisé en ce que** le piston (30) bascule autour d'un axe de pivotement (66).

15. Dispositif selon une ou plusieurs des revendications 1 à 14, **caractérisé en ce que** le fond de la chambre de transport, de la chambre de mesure (16) jusqu'à l'orifice de sortie (46), présente au moins localement une inclinaison d'au moins 30° lorsque la machine de récolte est à plat.

16. Dispositif de mesure de l'humidité du grain dans des machines de récolte comprenant une chambre de mesure, des moyens pour prélever le produit à mesurer sur un flux de produit récolté et pour amener le produit à mesurer dans la chambre de mesure, un détecteur d'humidité et des moyens pour ramener le produit à mesurer dans le flux de produit récolté-, **caractérisé en ce que** la chambre de mesure (16) est au moins partiellement disposée dans le flux de produit récolté et que des parois (60) d'une roue cellulaire (56) délimitent au moins partiellement la chambre de mesure (16).

17. Dispositif selon une ou plusieurs des revendications 1 à 16, **caractérisé en ce que** le point de retour du produit à mesurer dans le flux de produit récolté est adapté au cycle de mesure, de telle sorte qu'aucune fraction ou seulement une petite fraction de vieux produit récolté retourne dans la chambre de mesure.

18. Dispositif selon une ou plusieurs des revendications 1 à 17, **caractérisé en ce que** le point de retour du produit à mesurer débouche dans le tronçon d'un dispositif de transport (2), dans lequel il n'y a habituellement pas ou que peu de produit récolté

19. Dispositif selon une ou plusieurs des revendications 1 à 18, **caractérisé en ce qu'**après le nettoyage ou la vidange de la chambre de mesure (16), la valeur de mesure du détecteur d'humidité (18) est réglée sur une valeur décalée adaptée.
